# EUROPÄISCHE PATENTANMELDUNG

(11) **EP 1 132 464 A2**
(43) Veröffentlichungstag der Anmeldung: **12.09.2001**
(21) Anmeldenummer: 01105319.6
(22) Anmeldetag: 07.03.2001
(51) Int. Cl.: C12N 1/20, C12N 1/38, C02F 3/00

(54) **Verfahren zur Leistungssteigerung mikrobieller Systeme**

(30) Priorität: 10.03.2000 DE 10011728
(71) Anmelder: Schmid, Andreas, Dr., 95703 Plössberg (DE)
(72) Erfinder: Schmid, Andreas, Dr., 95703 Plössberg (DE)
(74) Vertreter: Motsch, Andreas

(57) **Zusammenfassung**

Es wird ein Verfahren zur Leistungssteigerung der Protein-Syntheserate eines mikrobiellen Systems vorgeschlagen, wobei eine äußere, zyklische Stressbeaufschlagung auf dieses System einwirkt und die Intervalle aus Stressbeaufschlagung und anschließender Ruhephase jeweils weniger als 45 Minuten betragen.

## Beschreibung

Die Erfindung betrifft ein Verfahren zur Leistungssteigerung der Protein-Syntheserate eines mikrobiellen Systems.

Von 1960 bis etwa 1980 wurden die molekularbiologischen Regulationsmechanismen bei prokaryontischen Organismen eruiert. Diese fanden in den letzten zehn Jahren wiederum verstärktes Interesse.

Es ist allgemein bekannt, dass in Mikroorganismen Regulationsmechanismen zum Tragen kommen, die die Koordination der Stoffwechselabläufe maßgebend bestimmen. Sie ermöglichen unter anderem die Ausnutzung spezifischer Nährsubstrate und damit die Anpassung an bestimmte Lebensräume. Es gibt mindestens zwei Niveaus, auf denen die Biosynthese der Proteine reguliert wird. Das eine ist die Kontrolle der Transkription, d. h. die Regulation der Transkription der DNA in mRNA und das andere ist die Kontrolle der Translation, d.h. die Regulation der Initiation und der Syntheserate der Polypeptidketten. Der größte Einfluss auf die Substrateliminationsrate von Mikroorganismen zeigt sich in den Änderungen der intra- und extrazellulären Enzymkonzentrationen durch Induktions- und Repressions-Mechanismen der Proteinsynthese innerhalb der Zelle (Schlegel H.G., Allgemeine Mikrobiologie, 7. Auflage, Springer, Berlin, Heidelberg, 1992). Die Modellierung der Proteinsynthese bei prokaryontischen Mikroorganismen stützt sich auf die grundlegenden Arbeiten von Jacob & Monod, Genetic Regulatory Mechanisms in the Synthesis of Proteins, Journal of Molecular Biology, 3, 318-356, 1961, die sich mit den genetischen Regulationsmechanismen befassen.

Darauf aufbauend zeigten verschiedene Wissenschaftler, dass nach Zugabe eines Induktors, z. B. eines Substrats, ein Überschwingverhalten der induzierten Proteinkonzentration beobachtet werden kann, das jedoch nach wenigen Minuten wieder auf ein stabiles Niveau abfällt (Goodwin B., Oscillatory Behaviour in Enzymatic Control Processes, Advances in Enzyme Regulation, 3, 425-438, 1965; Imanaka T., Kaieda T., Sato K., Taguchi H., Optimization of α-Galactosidase Production by Mold, Journal of Fermentation Technology, 50, 633-646, 1972; Knorre W., Theoretische und experimentelle Untersuchungen zur Biophysik von Regulationsprozessen, Leipzig, Univ. Diss., 1967; Murray J., Mathematical Biology, Springer Verlag Berlin, Heidelberg, 1989).

In oszillierenden Kulturen sahen J. Bailey, Periodic Operation of Chemical Reactors, A Review, Chemical Engineering Communication, 1, 111-124, 1973, und B. Sonnleitner, New Concepts for quantitative Bioprocess Research and Development, Advances in Biochemical Engineering and Biotechnology, 54, 155-188, 1996, Wachstumsvorteile, insbesondere bei intrazellulären Limitationen, die durch makroskopische Beeinflussung hervorgerufen werden können, gegenüber einer kontinuierlichen Betriebsweise der Proteinsynthese eines biologischen Systems.

Bei biologischen Verfahren der Proteinsynthese des Standes der Technik wird die Prozessstabilität durch möglichst lang anhaltende Betriebsbedingungen unter Ausschluss äußerer Störeinflüsse gewährleistet (Pöpel H.: Einfluss der Betriebsführung von Abwasserbehandlungsanlagen auf die Ablaufgüte. Aus: Betrieb von Abwasserbehandlungsanlagen, 36, Darmstädter Seminar Abwassertechnik, 13-39, 1994). Dies führte zur Entwicklung von kontinuierlich betriebenen Reaktoren als auch von Batch- und Semibatch-Reaktoren. Die äußere Einflussnahme auf Prozessparameter hierbei erstreckt sich auf einen Zeithorizont von mehr als einer Stunde, um die jeweils gewünschten Effekte zu erzielen (Wilderer P., Schroeder E.: Anwendung des Sequencing Batch Reactor (SBR)-Verfahrens zur biologischen Abwasserreinigung, Hamburger Berichte zur Siedlungswasserwirtschaft, 4, 1986; Metcalf & Eddy: Wastewater Engineering, Treatment, Disposal, Reuse. McGraw-Hill International Edition, 1991). Beispielhaft sei die intermittierende Belüftung zur Nitrifikation und Denitrifikation in einer biologischen Abwasserreinigungsstufe genannt. Veränderungen der Prozessparameter zu kürzeren Intervallen führte zu Instabilitäten, so dass Intervallperioden von weniger als 45 Minuten als nachteilig angesehen werden (Abwassertechnische Vereinigung: ATV-Handbuch - Biologische und weitergehende Abwasserreinigung, 4. Auflage, Ernst & Sohn Verlag, Berlin, 1977).

Es besteht jedoch ein grundsätzliches Bedürfnis zur Steigerung der Protein-Syntheserate von biologischen Systemen.

Der Erfindung liegt daher die Aufgabe zugrunde, ein Verfahren bereitzustellen, das zu einer Leistungssteigerung der Protein-Syntheserate eines mikrobiellen Systems führt.

Die Aufgabe wird durch ein Verfahren zur Leistungssteigerung der Protein-Syntheserate eines mikrobiellen Systems gelöst, das dadurch gekennzeichnet ist, dass eine äußere, zyklische Stressbeaufschlagung auf dieses System einwirkt und die Intervalle aus Stressbeaufschlagung und anschließender Ruhephase jeweils weniger als 45 Minuten betragen.

In dem erfindungsgemäßen Verfahren kann die Stressbeaufschlagung auf verschiedene Weise erfolgen:

Sie kann durch Konzentrationssprünge des induzierenden Substrats, wie Lactose, Allolactose zur Induktion von β-Galactosidase, oder des Substratanalogons wie Methyl-β-D-thiogalactosid zur Induktion von β-Galactosidase hervorgerufen werden.

Sie kann durch Konzentrationssprünge von Cofaktoren, wie Mineralstoffen, Vitaminen oder Spurenelementen hervorgerufen werden.

Sie kann durch Konzentrationssprünge von Effektoren, wie dem CAP-cAMP-Komplex bei der katabolischen Repression von ß-Galactosidase hervorgerufen werden. Hierbei bedeutet CAP Katabolit-Aktivatorprotein und cAMP zyklisches 3'5'-Adenosin-Monophosphat.

Sie kann durch eine Variation der Durchflussrate hervorgerufen werden. Diese kann das 0,1- bis 0,9-fache der determinierenden maximalen Wachstumsrate für reinen Chemostatbetrieb oder das 1,2- bis 4-fache der determinierenden maximalen Wachstumsrate für Chemostatbetrieb mit Biomasserezirkulation betragen.

Sie kann durch Änderung des pH-Werts hervorgerufen werden. Diese Veränderung bewegt sich im Bereich von bis 2 pH-Wert-Einheiten um den optimalen pH-Wert der determinierenden Mikroorganismen.

Sie kann durch eine Änderung der osmotischen Verhältnisse hervorgerufen werden, wobei diese bis zum 4-fachen der optimalen Osmolarität der determinierenden Mikroorganismen betragen kann.

Sie kann durch Temperaturänderung hervorgerufen werden, wobei diese bis 20° C um den optimalen Temperaturbereich der determinierenden Mikroorganismen betragen kann.

Bei den Regulationsmechanismen der katabolischen Enzymsynthese kommt es, wie erwähnt, zu Oszillationen der intra- und extrazellulären Enzymkonzentration. Diese Regulationsvorgänge spielen in mikrobiellen Systemen eine große Rolle, wobei jedoch das Überschwingverhalten der Proteinkonzentration bisher nicht für technische Zwecke genutzt werden konnte. Die mikrobiellen Reaktoren wurden unter stabilen äußeren Bedingungen betrieben mit Intervallperioden von mehr als einer Stunde, so dass sich ein Überschwingverhalten der induzierten Proteinkonzentration, das durch die genetischen Regulationsmechanismen hervorgerufen wurde, kaum merklich auf die vorliegende Proteinkonzentration, z. B. Enzymkonzentration, im Reaktor auswirkte. Daher konnten mögliche Leistungsreserven mikrobieller Systeme bisher nicht ausgeschöpft werden.

Erst durch das erfindungsgemäße Verfahren wird nunmehr die Nutzung des Überschwingverhaltens der Proteinkonzentration während der Induktions- und Repressionsphase technisch nutzbar. Wirkt erfindungsgemäß auf eine mikrobielle Population eine zyklische Stressbeaufschlagung in Intervallen von weniger als jeweils 45 Minuten ein, so treten die Regulationsmechanismen der Proteinsynthese mit ihrem zeitlichen Überschwingverhalten und Oszillationen stärker hervor und beeinflussen die real vorliegende Protein-Syntheserate. Durch geeignete Wahl der äußeren Stressintervalle kann nunmehr eine erhöhte Nettoproduktionsrate von vorwiegend katabolischen Enzymen erreicht werden.

Die genauen Zeitintervalle der zyklischen Stressbeaufschlagung werden von der zu stressenden Mikroorganismenspezies determiniert. Sie werden ferner von der Zusammensetzung, z.B. des Belebtschlamms, der zu stressenden Mikroorganismen-Mischpopulation, d.h. der Biozönose, determiniert.

Die Protein-Syntheseleistung wird zusätzlich durch die Durchflussrate und durch die induzierte Substratkonzentration determiniert.

Die angegebenen Abhängigkeiten lassen sich durch einfache Routineversuche jeweils optimieren.

Durch die erfindungsgemäßen zyklischen Änderungen der Prozessparameter in biologischen Reaktorsystemen können Wirkunsgradsteigerungen und erhöhte Stabilitäten bei der Produktbildung gegenüber äußeren Störeinflüssen in überraschender und vorteilhafter Weise erreicht werden.

Durch die spezifische Enzyminduktionsleistungssteigerung stellt sich ferner bei konstanter Biomassenkonzentration eine wesentlich größere Enzymkonzentration im Bioreaktor ein, als dies unter kontinuierlichen Bedingungen entsprechend dem Stand der Technik der Fall ist. Da der Umsatz beispielsweise bei mikrobiellen katabolischen Reaktionen direkt von der Enzymkonzentration abhängig ist, nimmt demzufolge auch der Wirkungsgrad zu. Daraus ergibt sich, dass unter dynamischen Prozessbedingungen in vorteilhafter Weise das notwendige Reaktionsvolumen drastisch reduziert werden kann.

Durch eine Prozessführung entsprechend vorliegender Erfindung können also die durch die Oszillationen hervorgerufenen Konzentrationsspitzen für technische Zwecke, wie beispielsweise eine erhöhte biologische Abbauleistung organischer Inhaltsstoffe in einer Kläranlage genutzt werden.

Die Anwendung vorliegender Erfindung führt zu einem besonders in technisch und wirtschaftlicher Hinsicht vorteilhaften Sanierungskonzept von existierenden Kläranlagen, da nunmehr ein Potenzial geschaffen wird, mögliche Erweiterungen einzusparen. Besonders im Hinblick auf Kläranlagengrößen bis ca.50000 EWG besteht Handlungsbedarf, da die vorherrschende EU-Richtlinie über die Behandlung kommunaler Abwässer entsprechende Erweiterungsmaßnahmen erforderlich machen.

Durch den erhöhten Selektionsdruck verbessert sich ferner die Ausbildung von spezialisierten und besonders robusten Mikroorganismenpopulationen, weshalb eine solch betriebene Kläranlage auch Problemstoffe wie beispielsweise Cyanide biochemisch mineralisieren kann.

Des weiteren ergeben sich weitreichende Anwendungsmöglichkeiten in der biologischen Abluftreinigung, Altlastensanierung, der pharmazeutischen wie auch chemischen Industrie beispielsweise bei der Enzymsynthese für die Waschmittelproduktion.

Ein weiterer Gegenstand der Erfindung besteht in einem der oben beschriebenen erfindungsgemäßen Verfahren, das dadurch gekennzeichnet ist, dass eine zeitliche Modulierung mit einer Frequenz bis zu mehreren Tagen der Intervalle der zyklischen Stressbeaufschlagung durchgeführt wird. Hierdurch wird eine langfristige Leistungssteigerung erzielt.

Die Modulierungsfrequenz wird von der zu stressenden Mikroorganismenspezies, von der Zusammensetzung der zu stressenden Mikroorganismen-Mischpopulation, von der Durchflussrate und von der induzierenden Substratkonzentration determiniert.

Ein weiterer Gegenstand der Erfindung besteht in einem der oben beschriebenen erfindungsgemäßen Verfahren, das dadurch gekennzeichnet ist, dass die Proteinsynthese mit mehrstufig-sequenziellen Protein-Induktionsabläufen durchgeführt wird. Hierdurch konnte eine Steigerung der Protein-Syntheseleistung um mindestens 20% erreicht werden.

Der Effekt der Protein-Synthese-Steigerung bei mehrstufig sequenziellen Proteininduktionsabläufen tritt z.B. bei katabolisch verlaufenden Induktionsabläufen schwer abbaubarer Stoffe auf.

Es wurden im Rahmen der Untersuchungen zu dem neuen Verfahren systemtheoretische Betrachtungen mit einem genetischen Modell durchgeführt. Hierbei zeigte sich eine mögliche Leistungssteigerung bei der Enzyminduktion von prokaryontischen Mikroorganismen unter zyklischer Beaufschlagung durch äußere Prozessparameter.

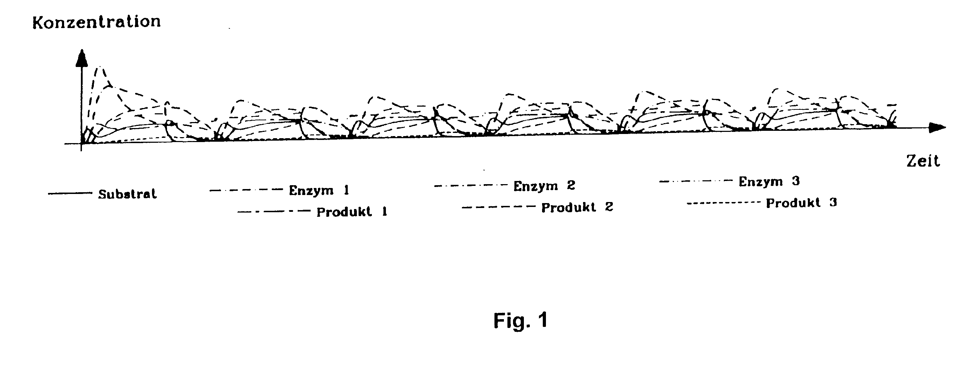

Die Figur 1 zeigt die Veränderung der Konzentrationen eines Substrats, von Enzymen 1, 2 und 3 sowie von Produkten 1, 2 und 3. Die Figur 1 stellt die Simulationsergebnisse des oben genannten genetischen Modells dar.

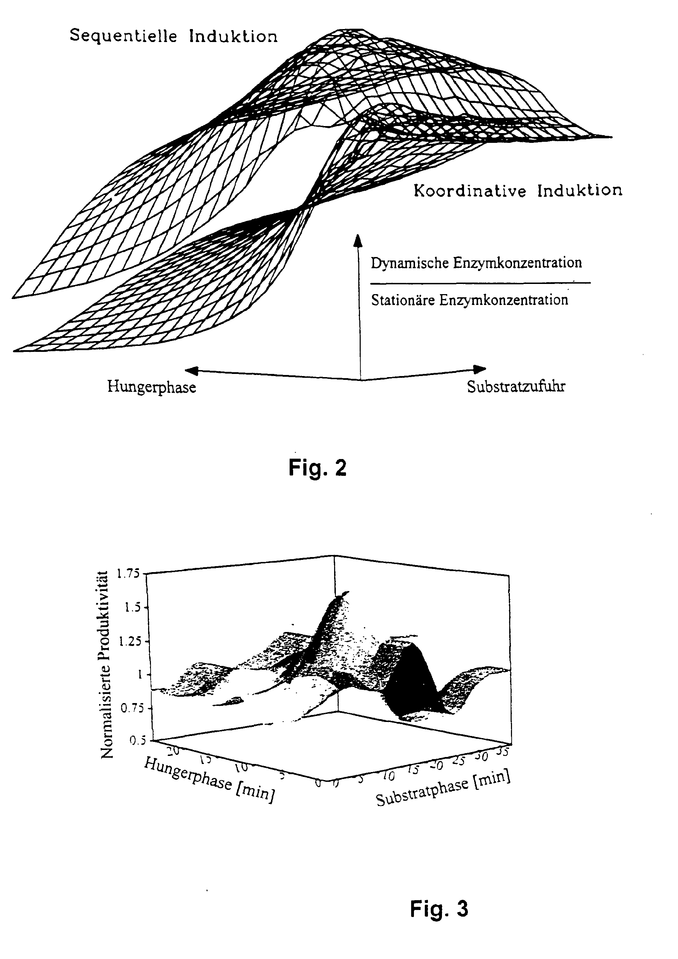

Die Figur 2 zeigt das Ergebnis einer Computersimulation für den Vergleich von koordinativer und sequenzieller Induktion. Daraus ergibt sich, dass eine höhere mittlere Enzymkonzentration bezogen auf das gleiche System bei der sequenziellen Induktion auftritt, da hier die Verzögerungszeiten zwischen Induktion und Repression wesentlich größer sind. Es zeigt sich ein biologisches Resonanzmuster.

Die Betriebsweise der sequenziellen Induktion ist deshalb besonders für mehrstufig-sequenzielle, katabolische Reaktionen, wie sie bei der Industrieabwasserreinigung auftreten, geeignet.

Die Erfindung soll nunmehr anhand eines Beispiels näher erläutert werden.

Die Untersuchung wurde in einem kontinuierlich durchflossenen, vollständig durchmischten Bioreaktor (Chemostat) mit Magermilchpulver, unter Zugabe von Nährsalzen, als Komplexsubstrat und Belebtschlamm-Inokulum ausgeführt. Das Komplexsubstrat wies hierbei eine zu kommunalem Abwasser ähnliche Charakteristik im Abbauverhalten auf. Der optimale Bereich zum biologischen Abbau des Magermilchpulver-Komplexsubstrats war durch eine periodische Substratdosierdauer von 15-21 Minuten und durch eine nachfolgende Hungerphase von 6-12 Minuten gekennzeichnet.

Die Figur 3 zeigt die Ergebnisse der Variationsversuche mit dem Chemostaten und dem synthetischen Komplexsubstrat, das mit kommunalem Abwasser vergleichbar ist. Hierbei ergab sich das dargestellte biologische Resonanzmuster mit einem Optimum der normalisierten Produktivität von mehr als 70 % über dem Wert bei kontinuierlicher Betriebsweise bei einer Substratdosier-Intervalldauer von 18 Minuten und einer nachfolgenden Hungerphase von 9 Minuten. Durch die zyklische Zugabe des Substrats in Minutenintervallen traten Konzentrationssprünge der Substratkonzentration im Reaktor auf, was die Mikroorganismen der Mischpopulation dazu stimuliert, verstärkt Enzyme zum Abbau des Ausgangssubstrats zu synthetisieren.

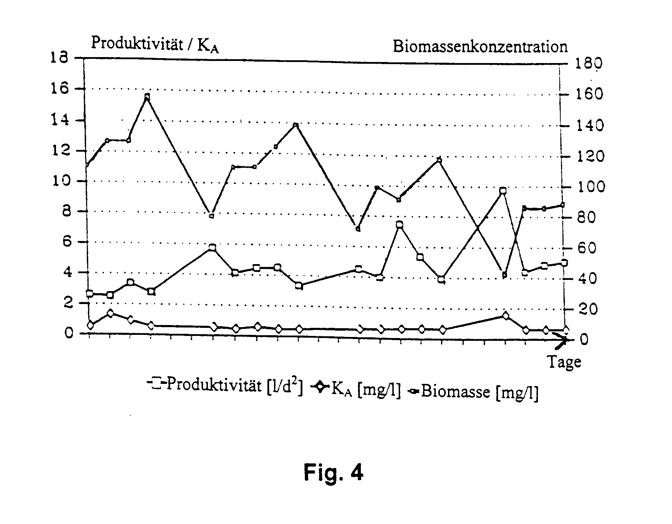

Die Figur 4 zeigt Kinetikparameter nach Biomasse-Inokulum unter optimierten Bedingungen. Das Verhalten der Biozönose wurde hierbei unter optimierten Bedingungen bei einer reduzierten Durchflussrate untersucht. In Figur 4 bedeutet K_{A} die Aktivitätskonstante, die ein Maß für die Affinität der genetischen Regulationsmechanismen zur Aktivierung der Proteinsynthese durch das induzierende Substrat darstellt. Die Substratzufuhr betrug 18, die Hungerphase 9 Minuten.

Wie in Figur 4 dargestellt, ergibt sich nach einer Erholungsphase von einigen Tagen eine erhöhte Produktivität mit einem Wert, der deutlich über dem Wert des stationären Niveaus mit einer Produktivität von 3,1 l/d² liegt.

Die in Figur 4 dargestellten Untersuchungen zeigen, dass das Resonanzphänomen unter optimierten Bedingungen auch langfristig Bestand hat. Dies ist eine wesentliche Voraussetzung für die technische Nutzung, da diese sich an einem dauerhaften Zustand erhöhter Produktivität eines mikrobiellen Systems orientieren muss.

Um ein langfristig hohes Niveau der Produktivität zu erreichen, wurde in weiteren Versuchsreihen bei Unterschreiten des Wertes von 4,0 l/d² das Hungerphasenintervall für den darauffolgenden Tag auf null Minuten reduziert, was eine kontinuierliche Beschickung bedeutet. Anschließend wurde wieder die optimierte Bedingung mit einem Hungerphasenintervall von 9 Minuten eingestellt. Es ergab sich eine im Durchschnitt um etwa 60% erhöhte Produktivität im Vergleich zu den stationären Bedingungen über mehrere Wochen hinweg bei dieser Verfahrensweise. Durch die zeitliche Variation der Substratzufuhr- und anschließenden Hungerphasenintervalle in Minutenbereichen kann also eine permanente Leistungssteigerung der Biozönose von etwa 60% auch über einen längeren Zeitraum hinweg erreicht werden. Die Optimierung des jeweiligen biologischen Reaktorsystems kann anhand von Routineversuchen erfolgen.

## Patentansprüche

1. Verfahren zur Leistungssteigerung der Protein-Syntheserate eines mikrobiellen, prokaryontischen und induzierbaren Systems **dadurch gekennzeichnet, dass** eine äußere, zyklische Stressbeaufschlagung auf dieses System einwirkt und die Intervalle aus Stressbeaufschlagung und anschließender Ruhephase jeweils weniger als 45 Minuten betragen.

2. Verfahren nach Anspruch 1, **dadurch gekennzeichnet, dass** die Intervalle aus Stressbeaufschlagung und anschließender Ruhephase jeweils 5 bis 30 Minuten betragen.

3. Verfahren nach Anspruch 2, **dadurch gekennzeichnet, dass** die Intervalle aus Stressbeaufschlagung und anschließender Ruhephase jeweils 6 bis 20 Minuten betragen

4. Verfahren nach einem der vorhergehenden Ansprüche, **dadurch gekennzeichnet, dass** die Stressbeaufschlagung durch Konzentrationssprünge des induzierenden Substrats hervorgerufen wird.

5. Verfahren nach einem der vorangehenden Ansprüche, **dadurch gekennzeichnet, dass** die Stressbeaufschlagung durch Konzentrationssprünge von Cofaktoren hervorgerufen wird.

6. Verfahren nach einem der vorhergehenden Ansprüche, **dadurch gekennzeichnet, dass** die Stressbeaufschlagung durch Konzentrationssprünge von Effektoren hervorgerufen wird.

7. Verfahren nach einem der vorhergehenden Ansprüche, **dadurch gekennzeichnet, dass** die Stressbeaufschlagung durch Variation der Durchflussrate hervorgerufen wird.

8. Verfahren nach einem der vorhergehenden Ansprüche, **dadurch gekennzeichnet, dass** die Stressbeaufschlagung durch Änderung des pH-Werts hervorgerufen wird.

9. Verfahren nach einem der vorhergehenden Ansprüche, **dadurch gekennzeichnet, dass** die Stressbeaufschlagung durch Änderung der osmotischen Verhältnisse hervorgerufen wird.

10. Verfahren nach einem der vorhergehenden Ansprüche, **dadurch gekennzeichnet, dass** eine zeitliche Modulierung mit einer Frequenz bis zu mehreren Tagen der Intervalle der zyklischen Stressbeaufschlagung durchgeführt wird.

11. Verfahren nach einem der vorhergehenden Ansprüche, **dadurch gekennzeichnet, dass** die Proteinsynthese mit mehrstufig-sequenziellen Protein-Induktionsabläufen durchgeführt wird.
